(19) Europäisches Patentamt
European Patent Office
Office européen des brevets

(11) **EP 4 725 389 A1**

(12) **EUROPEAN PATENT APPLICATION**
published in accordance with Art. 153(4) EPC

(43) Date of publication:
**15.04.2026 Bulletin 2026/16**

(21) Application number: **24819287.4**

(22) Date of filing: **03.06.2024**

(51) International Patent Classification (IPC):
*A61B 1/24* (2006.01)   *A61B 1/00* (2006.01)
*A61C 19/04* (2006.01)   *A61C 19/06* (2006.01)

(52) Cooperative Patent Classification (CPC):
A61B 1/00; A61B 1/24; A61C 19/04; A61C 19/06

(86) International application number:
**PCT/JP2024/020178**

(87) International publication number:
**WO 2024/253057 (12.12.2024 Gazette 2024/50)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **08.06.2023 JP 2023094470**

(71) Applicant: **Panasonic Intellectual Property Management Co., Ltd.**
**Kadoma-shi, Osaka 571-0057 (JP)**

(72) Inventors:
• **HAMASAKI, Takeshi**
  **Kadoma-shi, Osaka 571-0057 (JP)**
• **OHTSUKA, Yoshio**
  **Kadoma-shi, Osaka 571-0057 (JP)**

(74) Representative: **Novagraaf International SA**
**Chemin de l'Echo 3**
**1213 Onex, Geneva (CH)**

(54) **DENTAL PLAQUE DETECTION DEVICE, DENTAL PLAQUE DETECTION METHOD, AND PROGRAM**

(57)   A dental plaque detection device includes: an obtainer (101) that obtains a first image in which a blue-light region is at least partially attenuated from reflected light and fluorescence from a tooth, dental plaque, and a dental calculus inside an oral cavity that are being irradiated with irradiation light of a predetermined wavelength that excites a fluorescent substance contained in the dental plaque and the dental calculus; and a detector (102) that detects, from the first image, a content per unit area of the fluorescent substance contained in the dental plaque and the dental calculus adhering to the tooth. For instance, the detector (102) may generate an HSV image from the first image, and detect, from the value of the Value of the HSV image, the content per unit area of the fluorescent substance contained in the dental plaque and the dental calculus adhering to the tooth.

FIG. 5

## Description

[Technical Field]

The present disclosure relates to a dental plaque detection device, a dental plaque detection method, and a program.

[Background Art]

Patent Literature (PTL) 1 discloses a device that detects dental plaque on the basis of an image showing a tooth inside an oral cavity.

[Citation List]

[Patent Literature]

[PTL 1] Japanese Unexamined Patent Application Publication No. 2013-248220

[Summary of Invention]

[Technical Problem]

Such dental plaque detection devices are required to be able to detect a dental plaque condition in more detail.
In view above this, the present disclosure provides a dental plaque detection device or a dental plaque detection method by which it is possible to detect a tooth condition in detail.

[Solution to Problem]

A dental plaque detection device according to an aspect of the present disclosure includes: an obtainer that obtains a first image in which a blue-light region is at least partially attenuated from reflected light and fluorescence from a tooth, dental plaque, and a dental calculus inside an oral cavity that are being irradiated with irradiation light of a predetermined wavelength that excites a fluorescent substance contained in the dental plaque and the dental calculus; and a detector that detects, from the first image, a content per unit area of the fluorescent substance contained in the dental plaque and the dental calculus adhering to the tooth.

[Advantageous Effects of Invention]

The present disclosure can provide a dental plaque detection device or a dental plaque detection method by which it is possible to detect a tooth condition in detail.

[Brief Description of Drawings]

[FIG. 1]

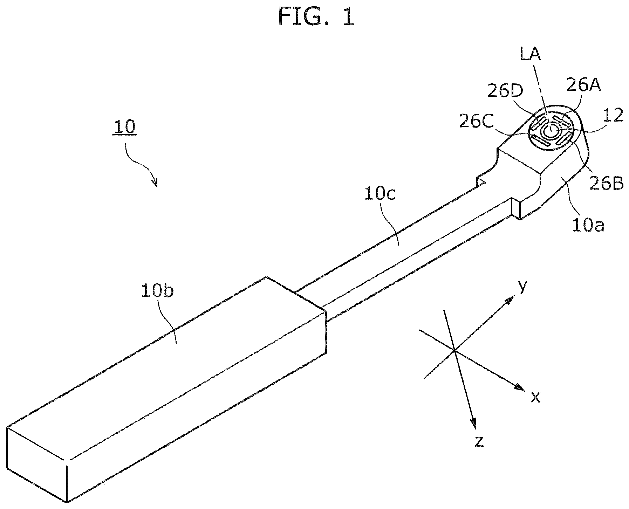

FIG. 1 is a perspective view of the intraoral camera of an intraoral camera system according to an embodiment.
[FIG. 2]

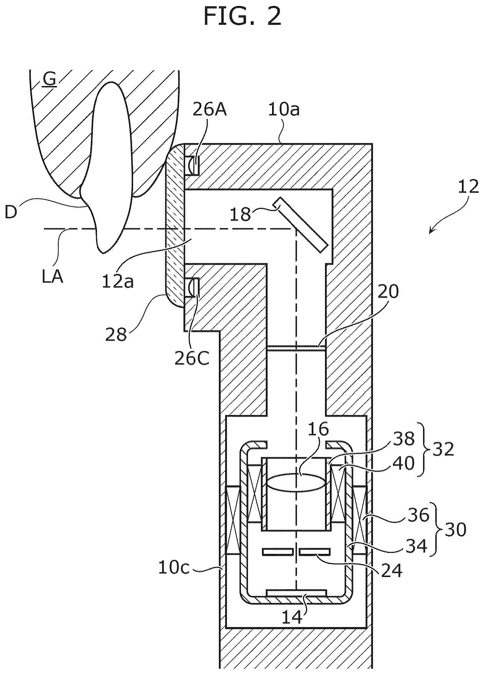

FIG. 2 is a cross-sectional view schematically illustrating an imaging optical system incorporated into the intraoral camera of the intraoral camera system according to the embodiment.
[FIG. 3]

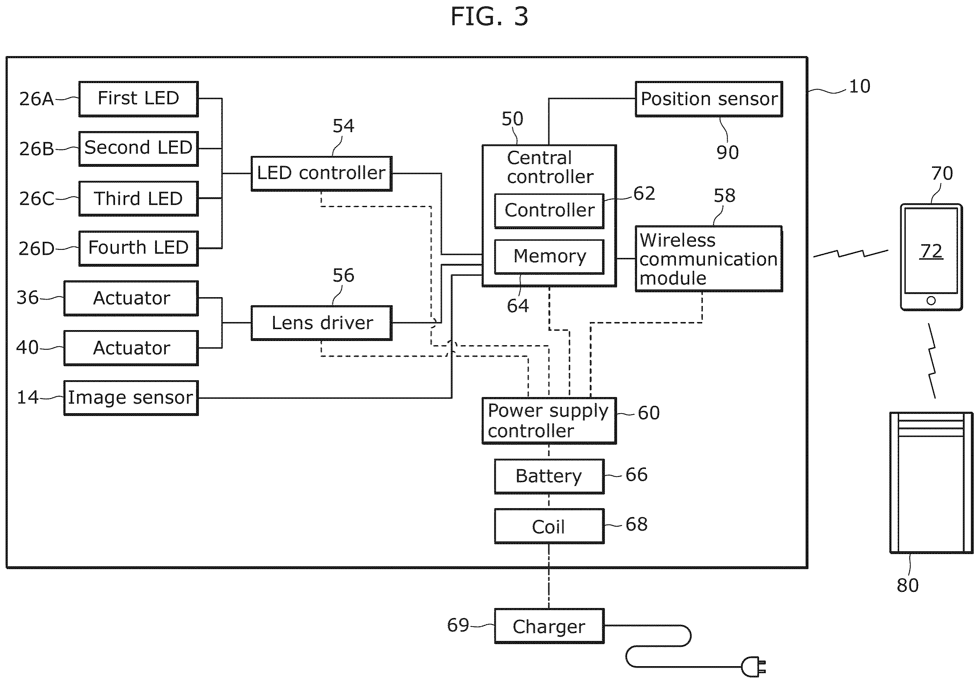

FIG. 3 illustrates a schematic configuration of the intraoral camera system according to the embodiment.
[FIG. 4]

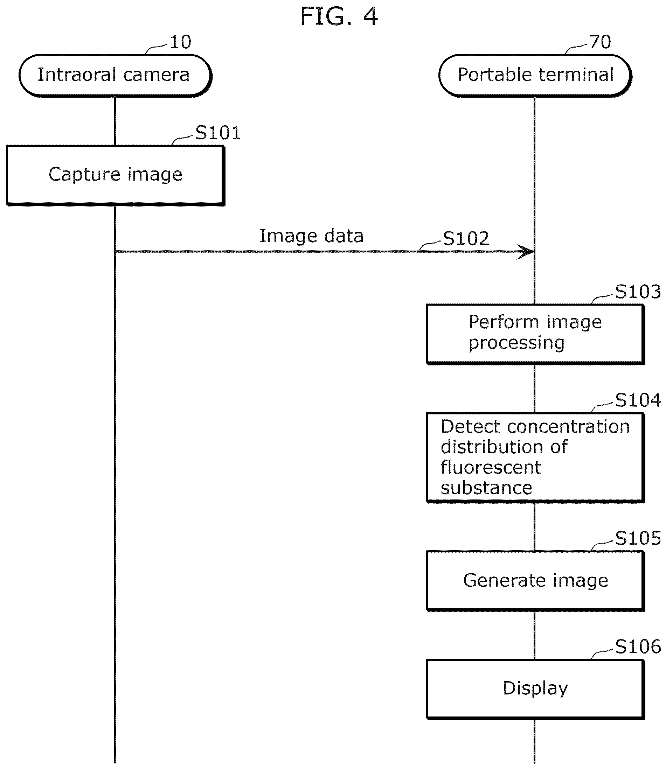

FIG. 4 illustrates a procedure of the operation of the intraoral camera system according to the embodiment.
[FIG. 5]

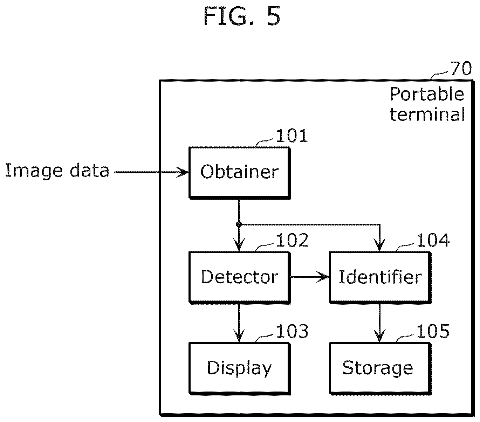

FIG. 5 is a functional block diagram of a portable terminal according to the embodiment.
[FIG. 6]

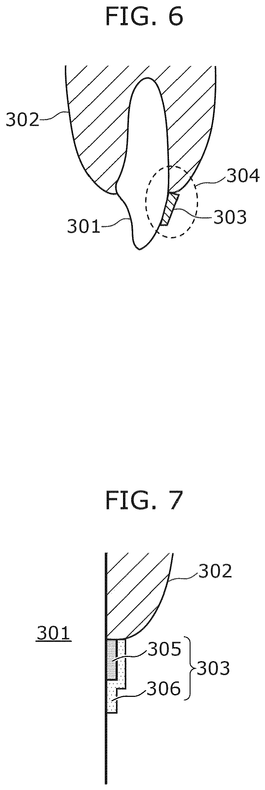

FIG. 6 illustrates an example of a tooth inside an oral cavity according to the embodiment.
[FIG. 7]

FIG. 7 illustrates an example of a layered model according to the embodiment.

[FIG. 8]

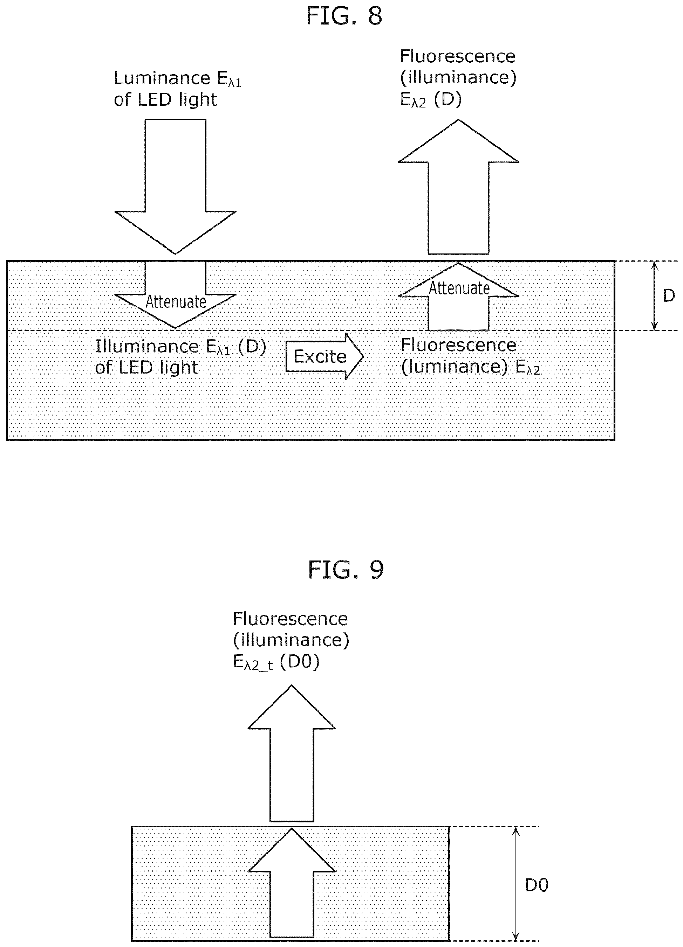

FIG. 8 is a figure for explaining the illuminance of fluorescence at depth D according to the embodiment.

[FIG. 9]

FIG. 9 is a figure for explaining the illuminance of fluorescence observed in dental plaque of thickness D0 according to the embodiment.

[FIG. 10]

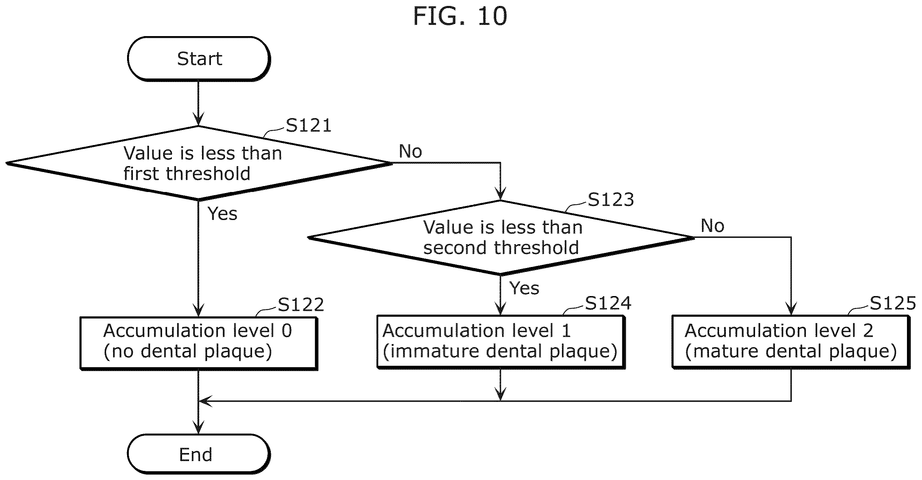

FIG. 10 is a flowchart illustrating processing for detecting the concentration distribution of a fluorescent substance according to the embodiment.

[FIG. 11]

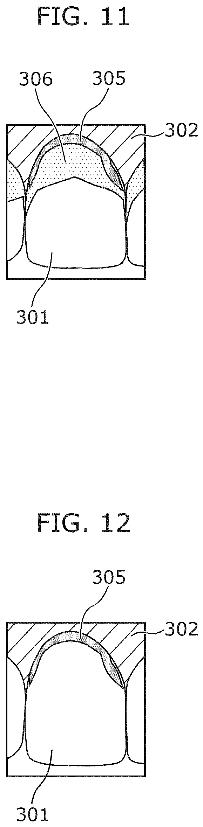

FIG. 11 illustrates an example of a fourth RGB image according to the embodiment.

[FIG. 12]

FIG. 12 illustrates an example of the fourth RGB image according to the embodiment.

[FIG. 13]

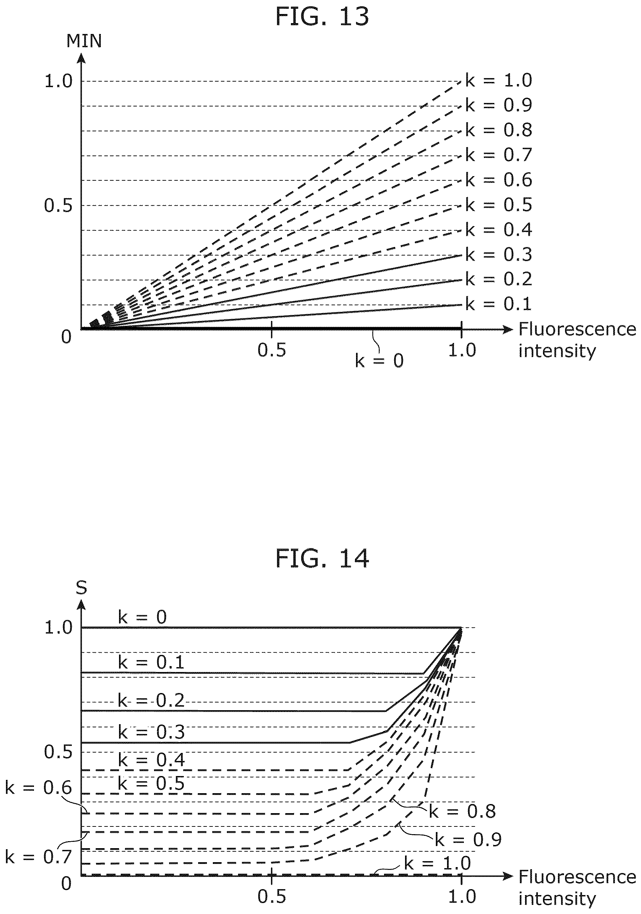

FIG. 13 illustrates a relationship between the fluorescence intensity, MIN, and k according to the embodiment.

[FIG. 14]

FIG. 14 illustrates a relationship between the fluorescence intensity, S (saturation), and k according to the embodiment.

[FIG. 15]

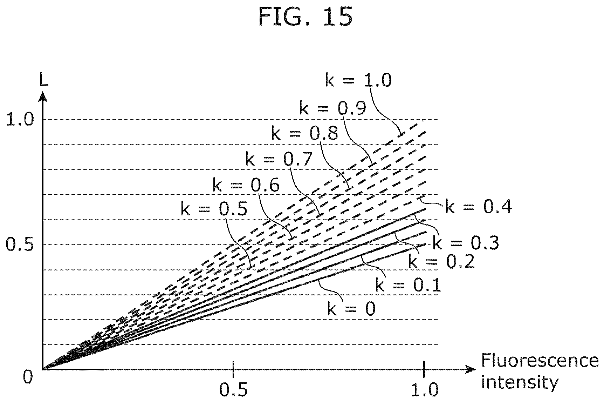

FIG. 15 illustrates a relationship between the fluorescence intensity, L (luminance), and k according to the embodiment.

[FIG. 16]

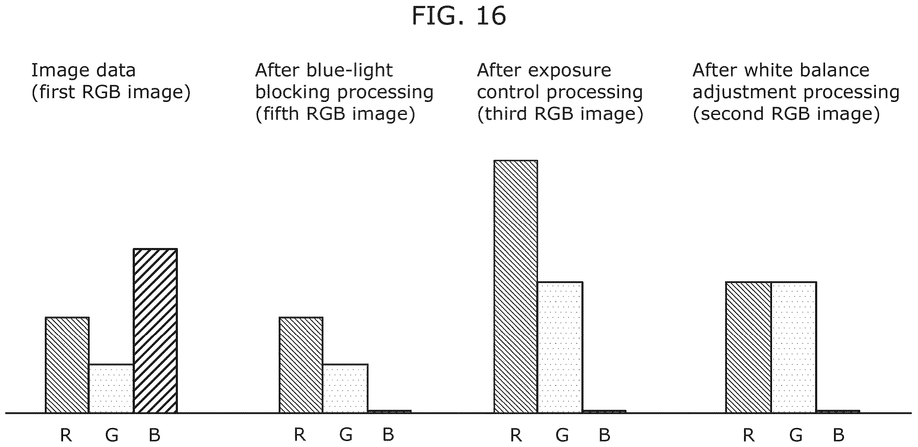

FIG. 16 illustrates an example of the pixel values of each image when a blue-light region is attenuated through signal processing according to the embodiment.

[FIG. 17]

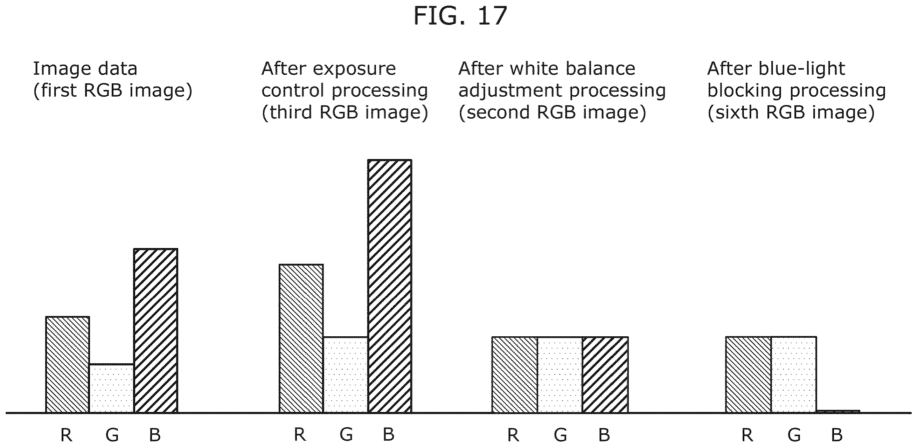

FIG. 17 illustrates an example of the pixel values of each image when a blue-light region is attenuated through the signal processing according to the embodiment.

[Description of Embodiment]

A dental plaque detection device according to one aspect of the present disclosure includes: an obtainer that obtains a first image in which a blue-light region is at least partially attenuated from reflected light and fluorescence from a tooth, dental plaque, and a dental calculus inside an oral cavity that are being irradiated with irradiation light of a predetermined wavelength that excites a fluorescent substance contained in the dental plaque and the dental calculus; and a detector that detects, from the first image, a content per unit area of the fluorescent substance contained in the dental plaque and the dental calculus adhering to the tooth. As such, the dental plaque detection device can detect the condition of the tooth in detail since the dental plaque detection device can detect the content per unit area of the fluorescent substance contained in the dental plaque and the dental calculus adhering to the tooth. Furthermore, the dental plaque detection device can improve the detection accuracy of the content per unit area of the fluorescent substance contained in the dental plaque and the dental calculus adhering to the tooth, by using the first image in which the blue-light region is at least partially attenuated from the reflected light and the fluorescence from the tooth, the dental plaque, and the dental calculus inside the oral cavity that are being irradiated with the irradiation light of the predetermined wavelength that excites the fluorescent substance contained in the dental plaque and the dental calculus.

For instance, the detector may generate an HSV image from the first image, and detect, from the value of the Value of the HSV image, the content per unit area of the fluorescent substance contained in the dental plaque and the dental calculus adhering to the tooth. Thus, the dental plaque detection device can detect the content per unit area of the fluorescent substance contained in the dental plaque and the dental calculus adhering to the tooth according to the value of the Value of the HSV image with high accuracy.

For instance, the first image may be a first RGB image, the detector may generate a second RGB image by performing, on the first RGB image, image processing including first image processing, in the first image processing, a gain for each of at least two color components among the red component, the green component, and the blue component of an RGB image to be processed may be adjusted so as to equalize the first red pixel average value of red pixel values, the first green pixel average value of green pixel values, and the first blue pixel average value of blue pixel values, the red pixel values, the green pixel values, and the blue pixel values being pixel values of a plurality of first pixels making up the portion of the RGB image to be processed that corresponds to the tooth, and the detector may generate the HSV image by converting the color space of the second RGB image into an HSV space.

Thus, by performing the first image processing, the dental plaque detection device can adjust the white balance of the first RGB image showing the fluorescing tooth. In this way, the dental plaque detection device can generate the second RGB image in which a dental plaque area of a tooth that is a portion to which dental plaque is adhering is easily distinguished. Thus, the dental plaque detection device can improve the detection accuracy of the content per unit area of the fluorescent substance contained in the dental plaque and the dental calculus adhering to the tooth.

For instance, the detector may identify a specific pixel area where one or more fourth pixels among the plurality of fourth pixels of the HSV image are located, the one or more fourth pixels satisfying at least one of the following: Saturation is within a first predetermined range; Hue is within a second predetermined range; and Value is within a third predetermined range, and the detector may detect, from the value of the Value of the specific pixel area, the content per unit area of the fluorescent substance contained in the dental plaque and the dental calculus adhering to the tooth. As such, the dental plaque detection device identifies a dental plaque area in a tooth image and then detects the content per unit area of the fluorescent substance contained in the dental plaque and the dental calculus adhering to the tooth, which enables the improvement in the detection accuracy of the content per unit area.

For instance, the detector may generate an HSL image from the first image, and detect, from the value of the luminance of the HSL image, the content per unit area of the fluorescent substance contained in the dental plaque and the dental calculus adhering to the tooth. As such, the dental plaque detection device can detect the content per unit area of the fluorescent substance contained in the dental plaque and the dental calculus adhering to the tooth, on the basis of the value of the luminance of the HSL image with high accuracy.

For instance, the first image may be a first RGB image, the detector may generate a second RGB image by performing, on the first RGB image, image processing including first image processing, in the first image processing, a gain for each of at least two color components among the red component, the green component, and the blue component of an RGB image to be processed may be adjusted so as to equalize the first red pixel average value of red pixel values, the first green pixel average value of green pixel values, and the first blue pixel average value of blue pixel values, the red pixel values, the green pixel values, and the blue pixel values being pixel values of a plurality of first pixels making up the portion of the RGB image to be processed that corresponds to the tooth, and the detector may generate the HSL image by converting the color space of the second RGB image into an HSL space.

Thus, by performing the first image processing, the dental plaque detection device can adjust the white balance of the first RGB image showing the fluorescing tooth. In this way, the dental plaque detection device can generate the second RGB image in which a dental plaque area of a tooth that is a portion to which dental plaque is adhering is easily distinguished. Thus, the dental plaque detection device can improve the detection accuracy of the content per unit area of the fluorescent substance contained in the dental plaque and the dental calculus adhering to the tooth.

For instance, the detector may identify a specific pixel area where one or more fifth pixels among the plurality of fifth pixels of the HSL image are located, the one or more fifth pixels satisfying at least one of the following: Saturation is within a fourth predetermined range; Hue is within a fifth predetermined range; and Luminance is within a sixth predetermined range, and the detector may detect, from the value of the Luminance of the specific pixel area, the content per unit area of the fluorescent substance contained in the dental plaque and the dental calculus adhering to the tooth. As such, the dental plaque detection device identifies a dental plaque area in a tooth image and then detects the content per unit area of the fluorescent substance contained in the dental plaque and the dental calculus adhering to the tooth, which enables the improvement in the detection accuracy of the content per unit area.

For instance, the fluorescent substance may be porphyrin. For instance, the detector may assign one of three or more gradation levels to each of contents per unit area of the fluorescent substance contained in the dental plaque and the dental calculus adhering to the tooth, and the detector may generate a third image in which the contents per unit area shown in gradation are superimposed on a second image based on the first image. As such, for instance, by using the generated third image, it is possible to notify a user of the contents per unit area of the fluorescent substance contained in the dental plaque and the dental calculus adhering to the tooth.

For instance, the dental plaque detection device may further include: an identifier that identifies the type of a tooth imaged; and storage that stores, in association with the type of the tooth identified, a content per unit area of a fluorescent substance contained in dental plaque and a dental calculus adhering to the tooth imaged, the content per unit area having been detected from the tooth imaged. As such, the dental plaque detection device can manage the content per unit area of the fluorescent substance contained in the dental plaque and the dental calculus adhering to each tooth.

In addition, a dental plaque detection method according to another aspect of the present disclosure includes: obtaining a first image in which a blue-light region is at least partially attenuated from reflected light and fluorescence from a tooth, dental plaque, and a dental calculus inside an oral cavity that are being irradiated with irradiation light of a predetermined wavelength that excites a fluorescent substance contained in the dental plaque and the dental calculus; and detecting, from the first image, a content per unit area of the fluorescent substance contained in the dental plaque and the dental calculus adhering to the tooth. As such, the condition of the tooth can be detected in detail by the dental plaque detection method since the content per unit area of the fluorescent substance contained in the dental plaque and the dental calculus adhering to the tooth can be detected by the dental plaque detection method. Furthermore, the dental plaque detection method can improve the detection accuracy of the content per unit area of the fluorescent substance contained in the dental plaque and the dental calculus adhering to the tooth, by using the first image in which the blue-light region is at least partially attenuated from the reflected light and the fluorescence from the tooth, the dental plaque, and the dental calculus inside the oral cavity that are being irradiation with the irradiation light of the predetermined wavelength that excites the fluorescent substance contained in the dental plaque and the dental calculus.

In addition, a program according to still another aspect of the present disclosure is a program for causing a computer to execute the dental plaque detection method.

It should be noted that these general or specific aspects may be embodied as a system, a method, an integrated circuit, a computer program, or a computer-readable recording medium such as a CD-ROM or may be embodied as any combination of the system, the method, the integrated circuit, the computer program, and the recording medium.

Hereinafter, an embodiment is described in detail with reference to the drawings as necessary. However, redundant explanations may be omitted. For instance, detailed explanations for well-known matters and overlapping explanations for substantially the same elements may be omitted. This is to avoid redundant explanations and facilitate understanding by those skilled in the art.

It should be noted that the inventors provide the appended drawings and the explanations below in order to help those skilled in the art to fully understand the present disclosure. Thus, the drawings and the explanations do not intend to limit the subject matter recited in the claims.

[Embodiment]

FIG. 1 is a perspective view of the intraoral camera of an intraoral camera system according to an embodiment. As illustrated in FIG. 1, intraoral camera 10 includes a toothbrush-shaped case that can be handled by one hand. The case includes head 10a, handle 10b, and neck 10c. Head 10a is put inside the oral cavity of a user when a dentition image is captured. Handle 10b is designed to be held by the user. Neck 10c connects head 10a and handle 10b.

FIG. 2 is a cross-sectional view schematically illustrating imaging optical system 12 incorporated into intraoral camera 10. As illustrated in FIG. 2, in the embodiment, imaging optical system 12 of intraoral camera 10 is incorporated into head 10a and neck 10c. Imaging optical system 12 includes image sensor 14 and lens 16 disposed on optical axis LA thereof.

Image sensor 14 is an imaging device, such as a complementary metal-oxide-semiconductor (CMOS) sensor or a charge coupled device (CCD), and lens 16 forms an image of tooth D. Image sensor 14 outputs a signal (image data) corresponding to the formed image to an external device.

Lens 16 is, for example, a condenser lens, and when light from tooth D is incident on lens 16, lens 16 forms an image of tooth D onto image sensor 14. It should be noted that lens 16 may be one lens or a lens group made up of a plurality of lenses.

For the embodiment, imaging optical system 12 further includes mirror 18, blue-light blocking filter (blue-light blocking element) 20, and aperture 24. Mirror 18 reflects the image of tooth D toward lens 16. Blue-light blocking filter 20 is disposed between mirror 18 and lens 16. Aperture 24 is disposed between lens 16 and image sensor 14.

Mirror 18 is disposed on optical axis LA of imaging optical system 12 so as to reflect the image of tooth D which has passed through light entry port 12a of imaging optical system 12 toward lens 16.

Blue-light blocking filter 20 is a filter that blocks the light component of a blue wavelength included in light to be incident on image sensor 14. When teeth are irradiated with light including a blue-light wavelength range to detect dental plaque, as the light including the blue-light wavelength range is intensified to intensify the excitation fluorescence of the dental plaque, the entirety of a first RGB image takes on a blue tinge. In this state, a blue pixel value is dominant over a red pixel value and a green pixel value, which may decrease the effect of making it easier to identify a dental plaque area, which can be obtained by performing image processing (exposure control processing and white balance adjustment processing), which is described later. To deal with this, blue-light blocking filter 20 blocks, from light yet to enter image sensor 14, a portion of the light including the blue-light wavelength range.

Aperture 24 is a plate-like component having a through hole on optical axis LA of imaging optical system 12 and achieves a deep depth of focus. Thus, a point in the depth direction of an oral cavity can be brought into focus, and a dentition image with a clear outline can be obtained.

In addition, intraoral camera 10 includes first to fourth LEDs 26A to 26D as illumination devices that illuminate target tooth D during image capturing. First to fourth LEDs 26A to 26D are, for example, blue light-emitting diodes (LEDs). In addition, as illustrated in FIG. 1, for the embodiment, first to fourth LEDs 26A to 26D surround light entry port 12a. It should be noted that translucent cover 28 covering first to fourth LEDs 26A to 26D and light entry port 12a is provided on head 10a so as not to run short of illumination light due to, for example, gums G being in contact with first to fourth LEDs 26A to 26D. It should be noted that one or more of first to fourth LEDs 26A to 26D may be one or more white LEDs. By using one or more white LEDs as one or more of first to fourth LEDs 26A to 26D, it is possible to increase the brightness of the first RGB image, which can improve the balance between the blue pixel value and the red pixel value and the balance between the blue pixel value and the green pixel value.

Furthermore, for the embodiment, as illustrated in FIG. 2, intraoral camera 10 includes composition adjustment mechanism 30 and focus adjustment mechanism 32.

Composition adjustment mechanism 30 includes case 34 holding image sensor 14 and lens 16 and actuator 36 for moving case 34 in the direction in which optical axis LA extends. When actuator 36 adjusts the position of case 34, the angle of view is adjusted, that is, the size of a dentition whose image is to be formed onto image sensor 14 is adjusted. It should be noted that composition adjustment mechanism 30 automatically adjusts the position of case 34 so that the entirety of a tooth is included in a captured image, for example. In addition, in accordance with a user operation, composition adjustment mechanism 30 adjusts the position of case 34 so as to achieve the angle of view desired by the user.

Focus adjustment mechanism 32 is held inside case 34 of composition adjustment mechanism 30, and includes lens holder 38 holding lens 16 and actuator 40 for moving lens holder 38 in the direction in which optical axis LA extends. The focus is adjusted by actuator 40 adjusting the relative position of lens holder 38 relative to image sensor 14. It should be noted that focus adjustment mechanism 32 automatically adjusts the position of lens holder 38 so that a tooth positioned in the middle of an image being captured is brought into focus. In addition, focus adjustment mechanism 32 adjusts the position of lens holder 38 in accordance with a user operation.

It should be noted that the structural elements of imaging optical system 12 except for mirror 18 may be provided in handle 10b of intraoral camera 10.

The image output by image sensor 14 is an RGB image in which each of the pixels of the image includes RGB subpixels.

In addition, intraoral camera 10 includes first to fourth LEDs 26A to 26D as illumination devices that illuminate a target tooth during image capturing. First to fourth LEDs 26A to 26D are, for example, blue LEDs that emit blue light having a wavelength peak of 405 nm. It should be noted that first to fourth LEDs 26A to 26D may be light sources for emitting light including the blue-light wavelength range and are not limited to blue LEDs.

FIG. 3 illustrates a schematic configuration of the intraoral camera system according to the embodiment. As illustrated in FIG. 3, in overview, the intraoral camera system according to the embodiment captures a dentition image by using intraoral camera 10 and performs the image processing for the captured image.

As illustrated in FIG. 3, the intraoral camera system includes intraoral camera 10, portable terminal 70, and cloud server 80. Portable terminal 70 is, for example, a smartphone or a tablet terminal capable of performing wireless communication. Portable terminal 70 includes, as an input device and an output device, touch screen 72 capable of displaying, for example, the dentition image. Portable terminal 70 functions as the user interface of the intraoral camera system.

Cloud server 80 is a server capable of communicating with portable terminal 70 via, for example, the internet and provides portable terminal 70 with an application for using intraoral camera 10. For instance, the user downloads the application from cloud server 80 and installs the application on portable terminal 70. In addition, cloud server 80 obtains, via portable terminal 70, the dentition image captured by intraoral camera 10.

Intraoral camera 10 includes, as main parts that perform system control, central controller 50, LED controller 54, lens driver 56, and position sensor 90. Here, LED controller 54 controls LEDs 26A to 26D, and lens driver 56 controls actuator 36 of composition adjustment mechanism 30 and actuator 40 of focus adjustment mechanism 32.

In addition, intraoral camera 10 includes wireless communication module 58 that performs wireless communication with portable terminal 70 and power supply controller 60 that supplies power to, for example, central controller 50.

Central controller 50 of intraoral camera 10 is included in, for example, handle 10b of intraoral camera 10. For instance, central controller 50 includes controller 62 such as a central processing unit (CPU) or a micro processing unit (MPU) which performs various processing tasks described later and memory 64 such as random access memory (RAM) or read only memory (ROM) storing program(s) for causing controller 62 to perform the various processing tasks. It should be noted that memory 64 stores, for example, a dentition image (image data) captured by image sensor 14 and various setting data items, in addition to the program(s). The dentition image captured by image sensor 14 is an example of the first RGB image.

Controller 62 transmits the dentition image output by image sensor 14 to portable terminal 70 via wireless communication module 58. Portable terminal 70 displays the transmitted dentition image on touch screen 72 and thus presents the dentition image to the user.

LED controller 54 is included in, for example, handle 10b of intraoral camera 10 and switches on and off first to fourth LEDs 26A to 26D in accordance with a control signal from controller 62. LED controller 54 is, for example, a circuit. When for instance the user performs an operation to activate intraoral camera 10 on touch screen 72 of portable terminal 70, a corresponding signal is transmitted from portable terminal 70 to controller 62 via wireless communication module 58. In accordance with the received signal, controller 62 transmits a control signal to LED controller 54 to cause LED controller 54 to switch on first to fourth LEDs 26A to 26D.

Lens driver 56 is included in, for example, handle 10b of intraoral camera 10 and controls actuator 36 of composition adjustment mechanism 30 and actuator 40 of focus adjustment mechanism 32 in accordance with a control signal from controller 62 of central controller 50. Lens driver 56 is, for example, a circuit. When for instance the user performs an operation related to composition adjustment or focus adjustment on touch screen 72 of portable terminal 70, a corresponding signal is transmitted from portable terminal 70 to central controller 50 via wireless communication module 58. In accordance with the received signal, controller 62 of central controller 50 transmits a control signal to lens driver 56 to cause lens driver 56 to perform the composition adjustment or the focus adjustment. In addition, for instance, in accordance with the dentition image received from image sensor 14, controller 62 calculates the amount of control for actuator 36 or actuator 40 required for the composition adjustment or the focus adjustment. Then, a control signal corresponding to the calculated amount of control is transmitted to lens driver 56.

Wireless communication module 58 is included in, for example, handle 10b of intraoral camera 10 and performs wireless communication with portable terminal 70 in accordance with the control signal from controller 62. Wireless communication module 58 performs, with portable terminal 70, wireless communication complying with an existing communication standard, such as Wi-Fi (registered trademark) or Bluetooth (registered trademark). A dentition image showing tooth D is transmitted from intraoral camera 10 to portable terminal 70 via wireless communication module 58, and an operation signal is transmitted from portable terminal 70 to intraoral camera 10 via wireless communication module 58.

For the embodiment, power supply controller 60 is included in handle 10b of intraoral camera 10 and distributes the power of battery 66 to central controller 50, LED controller 54, lens driver 56, and wireless communication module 58. Power supply controller 60 is, for example, a circuit. It should be noted that, for the embodiment, battery 66 is a rechargeable (secondary) battery and is wirelessly charged by external charger 69 connected to a commercial power source, via coil 68 included in intraoral camera 10.

Position sensor 90 is a sensor for detecting the orientation and the position of intraoral camera 10 and, for example, a multi-axis (here, X, Y, and Z-axis, that is, three-axis) acceleration sensor. For instance, position sensor 90 may be a six-axis sensor including a three-axis acceleration sensor and a three-axis gyro sensor. For instance, as illustrated in FIG. 1, the Z-axis matches optical axis LA. The Y-axis is parallel to the imaging plane and extends in a longitudinal direction of intraoral camera 10. In addition, the X-axis is parallel to the imaging plane and orthogonal to the Y-axis. Output by position sensor 90 for each axis may be transmitted to portable terminal 70 via central controller 50 and wireless communication module 58.

A piezo-resistive type, capacitive type, or heat detection type micro-electro-mechanical systems (MEMS) sensor may be used as position sensor 90. Although not illustrated in the figures, a correction circuit for correcting, for example, the balance of sensor sensitivity between the axes, the temperature characteristics of sensitivity, or temperature drift may be provided. In addition, a bandpass filter (a low pass filter) for removing dynamic acceleration components or noise may be provided. In addition, noise may be reduced by smoothing a waveform output by the acceleration sensor.

Then, the operation of the intraoral camera system is described. FIG. 4 illustrates a procedure of the operation of the intraoral camera system. It should be noted that the processing illustrated in FIG. 4 is, for example, processing performed in real time, and is performed for each frame or every time image data including a plurality of frames is obtained.

Image data is generated by the user capturing an image of teeth and gums inside their oral cavity with intraoral camera 10 (S101). The image data is, for example, image data obtained by capturing an image of a tooth fluorescing in response to exposure to light including a blue-light wavelength range. Then, intraoral camera 10 transmits the captured image data to portable terminal 70 (S102). It should be noted that the image data described here may be a video, one still image, or two or more still images. In addition, when the image data is a video or includes two or more still images, sensor data may be transmitted for each frame of the video or each still image. It should be noted that when the image data is a video, sensor data may be transmitted every two or more frames.

In addition, the image data may be transmitted in real time or transmitted at once after a series of image capturing (e.g., after capturing images of all the teeth inside the oral cavity).

Portable terminal 70 performs the image processing on the received image data (S103), and detects the concentration distribution of a fluorescent substance by using the image data that has undergone the image processing (S104). Then, portable terminal 70 generates an image in which the detected concentration distribution of the fluorescent substance is superimposed on the intraoral image (S105), and displays the generated image (S106).

By using such an intraoral camera system, the user can capture an intraoral image of the user with intraoral camera 10 and check the intraoral condition displayed on portable terminal 70. Furthermore, since the concentration distribution of the fluorescent substance is shown in the displayed image, the user can readily check their teeth's heath condition, for example.

In addition, portable terminal 70 may, for instance, generate the three-dimensional models of teeth inside the oral cavity from captured image data items. In addition, portable terminal 70 may display an image based on the generated three-dimensional models.

It should be noted that an example in which portable terminal 70 performs processing for a tooth image is described here. However, intraoral camera 10 may perform part of the processing or the whole processing. Portable terminal 70 is an example of a dental plaque detection device.

FIG. 5 is a functional block diagram of portable terminal 70. Portable terminal 70 includes obtainer 101, detector 102, display 103, identifier 104, and storage 105.

Obtainer 101 obtains the image data (the first RGB image) transmitted from intraoral camera 10. Obtainer 101 may obtain sensor data in addition to the image data from intraoral camera 10. The first RGB image is an image obtained by intraoral camera 10 capturing an image of teeth fluorescing in response to exposure to light including the blue-light wavelength range. Here, the blue light is an example of irradiation light of a predetermined wavelength that excites a fluorescent substance contained in dental plaque. In addition, the fluorescent substance is, for example, porphyrin.

Detector 102 may generate a third RGB image by performing the exposure control processing (second image processing) for the first RGB image, and generate a second RGB image by performing the white balance adjustment processing (first image processing) for the third RGB image.

(Exposure Control Processing)

In the exposure control processing, detector 102 first extracts a plurality of pixels whose RGB values satisfying the following Expressions 1 and 2 from among a plurality of first RGB pixels (third pixels) making up a first RGB image.

$$\min(R, G, B) \leq Ths, \text{ and, } \max(R, G, B) < Thmax \quad \text{(Expression 1)}$$

$$Gmax - G \leq Thb \qquad \text{(Expression 2)}$$

The smallest value among the pixel values of the three RGB subpixels of a first RGB pixel (that is, a red pixel value, a green pixel value, and a blue pixel value) is indicated by $\min(R, G, B)$.

Ths indicates a threshold for eliminating an area strongly affected by reflection of irradiation light (e.g., a glossy area), the area being a portion of the first RGB image. Ths is, for example, 900 in the 10-bit representation.

The largest value among the pixel values of the three RGB subpixels of the first RGB pixel (that is, the red pixel value, the green pixel value, and the blue pixel value) is indicated by $\max(R, G, B)$.

Thmax indicates the largest value that the pixel values can take. Thmax is expressed as, for example, 1023 in the 10-bit representation. Thmax is an example of a first threshold.

Gmax indicates the largest value among green pixel values included in the first RGB image. That is, Gmax is the pixel value of a green pixel having the largest pixel value among the green pixels of the plurality of first RGB pixels making up the first RGB image. Thb indicates a threshold for extracting the green pixels of second pixels from among the plurality of first RGB pixels. The image becomes too bright as the value of Thb increases. Thus, for instance, Thb is set to a value lower than or equal to 10 in the 10-bit representation.

The glossy area is eliminated by using Expression 1, and a tooth area in the first RGB image is extracted by using Expression 2. That is, a plurality of pixels extracted by using Expressions 1 and 2 are a plurality of second pixels making up the tooth area. Thus, the plurality of second pixels are pixels, among the plurality of first RGB pixels (the third pixels) making up the first RGB image, that satisfy the following conditions: $\max(R, G, B)$, which indicates the largest pixel value among the pixel values of the color components, is lower than a first threshold (Thmax), and $\min(R, G, B)$, which indicates the smallest pixel value among the pixel values of the color components, is lower than or equal to a second threshold (Ths).

Detector 102 calculates the average value of the pixel values of the green pixels included in the plurality of second pixels, and determines a gain by which the pixel values of the three RGB subpixels are to be multiplied, according to the calculated average value of the pixel values of the green pixels. For instance, detector 102 determines, using the following Expression 3, the gain by which the pixel values of the three RGB subpixels are to be multiplied. The gain is obtained by dividing a target pixel value by the average value of the pixel values of the green pixels. Detector 102 generates a third RGB image by multiplying each of the plurality of first RGB pixels making up the first RGB image by the determined gain. More specifically, detector 102 generates the third RGB image by multiplying the pixel values of the three subpixels of each of the plurality of first RGB pixels by the determined gain. In other words, the pixel values of a plurality of third RGB pixels making up the third RGB image are pixel values obtained by multiplying the pixel values of the plurality of first RGB pixels making up the first RGB image by the determined gain. It should be noted that if a pixel value multiplied by the gain exceeds the largest value (1023 in the case of the 10-bit representation), detector 102 replaces the pixel value with 1023.

In the above explanation, the average value of the pixel values of the green pixels included in the plurality of second pixels extracted from the first RGB pixels by using Expression 2 is calculated, and then the gain by which the pixel values of the three RGB subpixels are to be multiplied is determined according to the calculated average value of the pixel values of the green pixels. However, determination of the gain is not limited to the above example. The average value of the pixel values of red pixels included in the plurality of second pixels extracted from the first RGB pixels may be calculated, and then the gain by which the pixel values of the three RGB subpixels are to be multiplied may be determined according to the calculated average value of the pixel values of the red pixels. Likewise, the average value of the pixel values of blue pixels included in the plurality of second pixels extracted from the first RGB pixels may be calculated, and then the gain by which the pixel values of the three RGB subpixels are to be multiplied may be determined according to the calculated average value of the pixel values of the blue pixels.

Thus, in the exposure control processing, a third RGB image is generated by determining a gain for the second-pixel values of the plurality of second pixels (pixels corresponding to the tooth area) included in an RGB image to be processed (here, the first RGB image) so as to set the average value of index values calculated from the second-pixel values to a predetermined value, and applying the determined gain to the first RGB pixel values of the plurality of first RGB pixels included in the first RGB image. It should be noted that an index value may be a value calculated from the pixel values of three RGB subpixels making up one pixel or may be one of the pixel values of the three RGB subpixels. Here, the average value of the index values is the average value of a color component having the largest pixel value among the red component, the green component, and the blue component of the first RGB image. In addition, the color component having the largest average value is a color component having the largest average value among the following three average values: the first red pixel average value of red pixel values, the first green pixel average value of green pixel values, and the first blue pixel average value of blue pixel values, the red pixel values, the green pixel values, and the blue pixel values being the pixel values of the plurality of first pixels making up the first RGB image. It should be noted that, the first red pixel average value, the first green pixel average value, and the first blue pixel average value need not be calculated or compared when determining the color component having the largest average value, and the color component having the largest average value may be fixed to the green component.

It should be noted that in the above example, in determining the gain, detector 102 calculates the average value of the pixel values of the green pixels included in the plurality of second pixels, and determines the gain according to the calculated average value of the pixel values of the green pixels. However, determination of the gain is not limited to the above example. Detector 102 may calculate the average value of the luminance values of the plurality of second pixels as the average value of the index values, and determine the gain according to the calculated average value of the luminance values. Thus, the index value may be one of the pixel values of three RGB subpixels making up one pixel or may be a value calculated from the pixel values of the three RGB subpixels. Specifically, detector 102 calculates the luminance value of each of the plurality of second pixels, using the subpixel values of the three subpixels of the second pixel. For instance, detector 102 calculates the luminance value by using the following Expression 3.

$$Y = 0.21*R + 0.72*G + 0.07*B \qquad (\text{Expression 3})$$

In Expression 3, Y indicates the luminance value, R indicates the red pixel value, G indicates the green pixel value, and B indicates the blue pixel value.

Thus, luminance values may be obtained based on the red pixel value, the green pixel value, and the blue pixel value included in each of pixel values.

(White Balance Adjustment Processing)

In the white balance adjustment processing, detector 102 extracts, from among the plurality of third RGB pixels making up the third RGB image to be processed, a plurality of pixels whose RGB values satisfying the following Expressions 1 and 4.

$$Thl \leq Y \leq Thu \qquad (\text{Expression 4})$$

In Expression 4, Thl indicates a threshold indicating the lower limit of the tooth area, and Thu indicates a threshold indicating the upper limit of the tooth area.

The tooth area in the third RGB image is extracted by using Expression 4. That is, a plurality of pixels extracted by using Expressions 1 and 4 are the plurality of second pixels making up the tooth area.

Then, detector 102 calculates first red pixel average value Rave that is the average value of the red pixel values in the tooth area satisfying Expressions 1 and 4, first green pixel average value Gave that is the average value of the green pixel values in the tooth area, and first blue pixel average value Bave that is the average value of the blue pixel values in the tooth area. Then, detector 102 adjusts a gain for each of at least two color components among the red component, the green component, and the blue component of an RGB image to be processed, so as to equalize first red pixel average value Rave, first green pixel average value Gave, and first blue pixel average value Bave.

Specifically, detector 102 calculates the gain for the red pixel values (the gain for red pixels) by dividing first green pixel average value Gave by first red pixel average value Rave. In addition, detector 102 calculates the gain for the blue pixel values (the gain for blue pixels) by dividing first green pixel average value Gave by first blue pixel average value Bave. Then, detector 102 generates a second RGB image by multiplying each of the red pixels of the plurality of third RGB pixels making up the third RGB image by the gain for the red pixels and multiplying each of the blue pixels of the plurality of third RGB pixels by the gain for the blue pixels. In other words, the pixel values of a plurality of second RGB pixels making up the second RGB image are pixel values obtained by multiplying the red pixel values of the plurality of third RGB pixels making up the third RGB image by the gain for the red pixels and multiplying the blue pixel values of the plurality of third RGB pixels by the gain for the blue pixels. It should be noted that in the above example, detector 102 adjusts the white balance by calculating the gain for the red pixels and the gain for the blue pixels by using the green pixel average value as a reference and multiplying, by each gain, the pixel values of a color component corresponding to the gain. However, using the green pixel average value as the reference is just an example. The gain for the green pixels and the gain for the blue pixels may be calculated using the red pixel average value as the reference. The gain for the red pixels and the gain for the green pixels may be calculated using the blue pixel average value as the reference.

It should be noted that if a pixel value multiplied by the gain exceeds the largest value (1023 in the case of the 10-bit representation), detector 102 replaces the pixel value with 1023.

In addition, detector 102 may emphasize a dental plaque area within the tooth area in the second RGB image by performing third image processing as described below for the second RGB image. Specifically, detector 102 generates an HSV image by converting the color space of the second RGB image into an HSV space. Then, detector 102 identifies, as the dental plaque area, a specific pixel area in which one or more fourth pixels among a plurality of fourth pixels of the HSV image are located, the one or more fourth pixels satisfying one of the following conditions: the saturation is within a first predetermined range (e.g., at least 30 and at most 80 in the 8-bit representation), the hue is within a second predetermined range (e.g., at least 140 and at most 170 in the 8-bit representation), and the value (brightness) is within a third predetermined range (e.g., at least 100 and at most 180 in the 8-bit representation). It should be noted that the first predetermined range, the second predetermined range, and the third predetermined range may be identified by comparing the actual dental plaque area and tooth area with the HSV image. The predetermined ranges are not limited to the above numerical value ranges.

It should be noted that the value ranges of saturation, hue, and value can be determined by comparing with the dyeing degree of dental plaque dyed by an applied dental plaque disclosing agent.

(Processing for Detecting Concentration Distribution of Fluorescent Substance)

Detector 102 detects the concentration distribution of a fluorescent substance by using an HSV image. Specifically, detector 102 detects the concentration distribution of the fluorescent substance by using the value of value V of the HSV image.

FIG. 6 illustrates an example of a tooth inside an oral cavity. FIG. 6 illustrates tooth 301, gingiva 302, and dental plaque 303. FIG. 7 illustrates an example in which the structure of area 304 illustrated in FIG. 6 is illustrated as a layered model. As illustrated in FIG. 7, in dental plaque 303, mature dental plaque (dental calculus) 305 and immature dental plaque 306 are stacked.

In the process of blue light transmitting through each layer, porphyrin in the dental plaque is excited, which generates red fluorescence. In addition, the fluorescence intensity is considered indicating the accumulation of the fluorescent substance (porphyrin), rather than reflecting the current bacterial flora. That is, the red fluorescence becomes darker with an increase in the accumulation of the fluorescent substance. This means that the accumulation level of porphyrin increases as the dental plaque becomes mature. Thus, the fluorescence intensity of mature dental plaque 305 is higher than that of immature dental plaque 306.

By comparing the intensity of the red fluorescence per unit area of the dental plaque area, detector 102 detects the accumulation level (concentration or density) of the fluorescent substance. That is, detector 102 detects the content per unit area of the fluorescent substance contained in dental plaque and a dental calculus adhering to the tooth.

As described above, the dental plaque area including the one or more fourth pixels is extracted from the HSV image, the one or more fourth pixels satisfying at least one of the following conditions: saturation S is within the first predetermined range, hue H is within the second predetermined range, and value V is within the third predetermined range.

In addition, when the largest value among the value of R, the value of G, and the value of B is defined as MAX, and the smallest value among the values is defined as MIN, the following Expressions 5, 6, and 7 hold in the cylindrical model of the HSV space.

[Math. 1]

$$H = \begin{cases} undefined, & if\ MIN = MAX \\ 60 \times \dfrac{G-R}{MAX-MIN} + 60, & if\ MIN = B \\ 60 \times \dfrac{B-G}{MAX-MIN} + 180, & if\ MIN = R \\ 60 \times \dfrac{R-B}{MAX-MIN} + 300, & if\ MIN = G \end{cases}$$

(Expression 5)

$$V = MAX$$ (Expression 6)

$$S = \frac{MAX - MIN}{MAX}$$ (Expression 7)

Here, the second RGB image is an image that has undergone the white balance adjustment processing. Thus, MAX = R, and MIN = G or B. That is, in the dental plaque area, value V is determined by the value of R regardless of saturation S or hue H.

In addition, it is known that a fluorescent substance, porphyrin contained in dental plaque has a fluorescence wavelength of 600 nm to 740 nm and has a peak fluorescence wavelength of 630 nm. That is, the concentration of porphyrin accumulated in the dental plaque area can be evaluated by detecting the value of value V of the HSV image of the dental plaque area.

In addition, a layered biofilm model can be used as the layered model illustrated in FIG. 7. FIG. 8 is a figure for explaining the illuminance of fluorescence at depth D.

In the biofilm model, LED light and fluorescence from the dental plaque advance through the dental plaque while attenuating. The attenuation of the LED light and the fluorescence from the dental plaque outside the dental plaque (that is, in the air) is negligible. The luminance of the fluorescence from the dental plaque is proportional to the illuminance of the LED light to which the dental plaque is exposed. The fluorescence from the dental plaque emits light from a plane exposed to the LED light toward a place from which the LED light is coming (in the reflection direction). As analysis of a depth direction, for simplicity's sake, we assume that the LED light and the fluorescence from the dental plaque are uniform plane light sources (that is, luminance = illuminance).

Specifically, LED light of luminance $E_{\lambda 1}$ is uniformly emitted. Dental plaque density is uniformly distributed, and attenuation rate $\sigma_{\lambda 1}$ of the LED light due to the dental plaque is constant regardless of depth D. In this case, illuminance $E_{\lambda 1}$ (D) of the LED light at depth D is expressed by Expression 8. Here, since spectral transmittance $T_{d\lambda 1}$ is expressed by Expression 9, Expression 10 is obtained from Expressions 8 and 9.

[Math. 2]

$$E_{\lambda 1}(D) = T_{d\lambda 1} \cdot E_{\lambda 1}$$ (Expression 8)

$$T_{d\lambda 1} = E_{\lambda 1} \cdot exp(-\sigma_{\lambda 1} \cdot D)$$ (Expression 9)

$$E_{\lambda 1}(D) = E_{\lambda 1} \cdot exp(-\sigma_{\lambda 1} \cdot D)$$ (Expression 10)

In addition, luminance $E_{\lambda 2}$ of the fluorescence from the dental plaque at depth D is expressed by Expression 11 using proportionality constant k.

[Math. 3]

$$E_{\lambda 2} = k \cdot E_{\lambda 1}(D) \qquad \text{(Expression 11)}$$

In addition, if depth D is the same, luminance $E_{\lambda 2}$ of the fluorescence that the dental plaque emits is the same regardless of the location. Dental plaque density is uniformly distributed, and attenuation rate $\sigma_{\lambda 2}$ of the fluorescence due to the dental plaque is constant regardless of depth D. In this case, illuminance $E_{\lambda 2}$ (D) of the fluorescence observed in the dental plaque at depth D is expressed by Expression 12. Here, since spectral transmittance $T_{d\lambda 2}$ is expressed by Expression 13, Expression 14 is obtained from Expressions 12 and 13. Furthermore, Expression 15 is obtained from Expressions 10, 11, and 14.

[Math. 4]

$$E_{\lambda 2}(D) = T_{d\lambda 2} \cdot E_{\lambda 2} \qquad \text{(Expression 12)}$$

$$T_{d\lambda 2} = E_{\lambda 2} \cdot exp(-\sigma_{\lambda 2} \cdot D) \qquad \text{(Expression 13)}$$

$$E_{\lambda 2}(D) = E_{\lambda 2} \cdot exp(-\sigma_{\lambda 2} \cdot D) \qquad \text{(Expression 14)}$$

$$E_{\lambda 2}(D) = k \cdot E_{\lambda 1} \cdot exp(-\sigma_{\lambda 1} \cdot D - \sigma_{\lambda 2} \cdot D)$$

$$= k \cdot E_{\lambda 1} \cdot exp\{-(\sigma_{\lambda 1} + \sigma_{\lambda 2}) \cdot D\} \qquad \text{(Expression 15)}$$

FIG. 9 is a figure for explaining illuminance $E_{\lambda 2\_t}$ (D0) of fluorescence observed in dental plaque of thickness D0. Illuminance $E_{\lambda 2\_t}$ (D0) of the fluorescence observed in the dental plaque of thickness D0 can be expressed as the integrated value of $E_{\lambda 2}(D)$. Specifically, $E_{\lambda 2\_t}$ (D0) is expressed by Expression 16.
[Math. 5]

$$E_{\lambda 2\_t}(D0) = \int_0^{D0} E_{\lambda 2}(D)\, dD$$

$$= \int_0^{D0} k \cdot E_{\lambda 1} \cdot exp\{-(\sigma_{\lambda 1} + \sigma_{\lambda 2}) \cdot D\}\, dD$$

$$= \frac{k \cdot E_{\lambda 1}}{-(\sigma_{\lambda 1} + \sigma_{\lambda 2})} \left[ exp\{-(\sigma_{\lambda 1} + \sigma_{\lambda 2}) \cdot D\} \right]_0^{D0}$$

$$= \frac{k \cdot E_{\lambda 1}}{-(\sigma_{\lambda 1} + \sigma_{\lambda 2})} [exp\{-(\sigma_{\lambda 1} + \sigma_{\lambda 2}) \cdot D0\} - exp\{-(\sigma_{\lambda 1} + \sigma_{\lambda 2}) \cdot 0\}]$$

$$= \frac{k \cdot E_{\lambda 1}}{-(\sigma_{\lambda 1} + \sigma_{\lambda 2})} [exp\{-(\sigma_{\lambda 1} + \sigma_{\lambda 2}) \cdot D0\} - 1]$$

$$= \frac{k \cdot E_{\lambda 1}}{-(\sigma_{\lambda 1} + \sigma_{\lambda 2})} \left[ 1 - \frac{1}{exp\{-(\sigma_{\lambda 1} + \sigma_{\lambda 2}) \cdot D0\}} \right] \qquad \text{(Expression 16)}$$

Thus, the luminance of red fluorescence from a dental plaque layer (biofilm) of thickness D0 reflects the accumulation level of porphyrin accumulated in the dental plaque layer (biofilm). It should be noted that in the above explanation, the porphyrin concentration within the dental plaque layer is constant. However, a fluorescence phenomenon can be explained with regard to the following case: the lower layer in the biofilm model contains a dental calculus and has a high porphyrin concentration, and the upper layer in the biofilm model contains immature dental plaque and has a low porphyrin concentration. That is, as illustrated in FIG. 7, when mature dental plaque 305 (a dental calculus) in the lower layer is covered with immature dental plaque 306 in the upper layer, the intensity of the red fluorescence of a portion where mature dental plaque 305 is present in the lower layer is higher than that of the red fluorescence of a portion where mature dental plaque 305 is not present in the lower layer.

In addition, it is known from Expression 16 that the fluorescence changes according to thickness D (the intensity of the fluorescence increases with an increase in thickness D). That is, the intensity of the fluorescence indicates the accumulation level proportional to the concentration and amount (thickness) of the fluorescent substance.

FIG. 10 is a flowchart illustrating processing for detecting the concentration distribution of the fluorescent substance performed by detector 102. For instance, detector 102 detects the accumulation level for each pixel by performing the processing illustrated in FIG. 10 on each pixel of a dental plaque area included in an HSV image. It should be noted that detector 102 may detect the accumulation level for each unit pixel including a plurality of pixels by performing the processing illustrated in FIG. 10 on each unit pixel. In this case, for instance, the average value of the Value of each of the plurality of pixels included in the unit pixel may be used.

First, detector 102 determines whether the value of a target pixel is less than a first threshold (S121). When the value of the target pixel is less than the first threshold (Yes in S121), detector 102 determines that the accumulation level of the target pixel is accumulation level 0 (for example, no dental plaque is present) (S122).

Meanwhile, when the value of the target pixel is greater than or equal to the first threshold (No in S121), detector 102 determines whether the value of the target pixel is less than a second threshold (S123). Here, the second threshold is greater than the first threshold. When the value of the target pixel is less than the second threshold (Yes in S123), that is, when the value of the target pixel is greater than or equal to the first threshold and less than the second threshold, detector 102 determines that the accumulation level of the target pixel is accumulation level 1 (for example, immature dental plaque) (S124).

Meanwhile, when the value of the target pixel is greater than or equal to the second threshold (No in S123), detector 102 determines that the accumulation level of the target pixel is accumulation level 2 (for example, mature dental plaque (a dental calculus)) (S125).

In this way, detector 102 detects the distribution of accumulation levels (the concentration of the fluorescent substance) by determining the accumulation level for each pixel. Here, the accumulation level distribution is information indicating the accumulation level for each two-dimensional position in the xy plane (for example, each pixel).

Next, detector 102 assigns different gradation levels to accumulation levels 0 to 2 (three accumulations levels), and generates a fourth RGB image in which for example the accumulation level distribution shown in gradation is superimposed on the second RGB image. FIG. 11 illustrates an example of the fourth RGB image. For instance, as illustrated in FIG. 11, the pattern of the first gradation level (for example, 0.5) is superimposed on an immature dental plaque 306 area, and the pattern of the second gradation level (for example, 1.0) is superimposed on a mature dental plaque 305 area.

It should be noted that although the example of the three accumulation levels is given above, the number of accumulation levels may be four or more. In addition, the second image on which the accumulation level distribution is to be superimposed may be an image other than the second RGB image. For instance, the second image may be the first RGB image or an image generated by performing image processing on the first RGB image or the second RGB image.

FIG. 12 illustrates an example of the tooth after performing oral care for the tooth with the condition illustrated in FIG. 11. As illustrated in FIG. 12, although immature dental plaque 306 has been removed by performing the oral care (e.g., tooth brushing), mature dental plaque 305 has not been removed.

As such, detector 102 may determine the score of the oral care (whether there is a portion that has not been brushed sufficiently) according to the condition of immature dental plaque 306. In addition, detector 102 may recommend the user to have a checkup with a dentist according to the condition of mature dental plaque 305.

For instance, detector 102 calculates the proportion of the area of the immature dental plaque 306 area in the area of the tooth area. That is, detector 102 calculates (the area of the immature dental plaque 306 area)/(the area of the tooth area) × 100% as the first area ratio that is the area ratio of immature dental plaque 306. In addition, detector 102 calculates the proportion of the area of the mature dental plaque 305 area in the area of the tooth area. That is, detector 102 calculates (the area of the mature dental plaque 305 area)/(the area of the tooth area) × 100% as the second area ratio that is the area ratio of mature dental plaque 305.

Detector 102 may determine the score of the oral care by using the calculated first area ratio. In addition, detector 102 may recommend the user to have a checkup with a dentist according to the calculated second area ratio. For instance, when the second area ratio is greater than a predetermined threshold, detector 102 may display a message that recommends the user to have a checkup with a dentist.

It should be noted that the calculation of the area ratio and the score determination may be performed all at once for all teeth inside the oral cavity. That is, the area ratio may be the ratio of the total area of the dental plaque (immature dental plaque 306 or mature dental plaque 305) to the total area of the tooth areas of all the teeth. Alternatively, the calculation of the area ratio and the score determination may be performed individually for each of teeth inside the oral cavity. That is, the area ratio may be the ratio of the area of dental plaque of one tooth to the area of the tooth area of the one tooth. Alternatively, the calculation of the area ratio and the score determination may be performed individually for each of teeth areas into which the teeth inside the oral cavity are divided. Each of the teeth areas is an area including two or more teeth, the area being an area such as a maxillary right posterior area or a mandibular left anterior area.

In addition, identifier 104 illustrated in FIG. 5 identifies the types of teeth in image data on the basis of the image data. Here, the type of a tooth is information by which it is possible to uniquely identify the tooth inside the oral cavity, such as a maxillary right central incisor or a mandibular left lateral incisor.

For instance, identifier 104 obtains reference data corresponding to the types of teeth from cloud server 80, and identifies the type of each tooth included in image data by, for example, comparing the amounts of features with the use of the image data and the obtained reference data.

Identifier 104 stores the concentration distribution (accumulation levels) of the fluorescent substance detected by detector 102 in storage 105 in association with the types of the teeth. That is, storage 105 stores the concentration distribution (accumulation levels) of the fluorescent substance for each tooth. In addition, the calculation of the area ratio and the score determination for each tooth may be performed using the information for each tooth.

Display 103 is the display device of portable terminal 70 and displays the fourth RGB image in which the accumulation level distribution is superimposed on the image data. In addition, display 103 displays, for example, the determination result and a message based on the determination result. In addition, display 103 may display, for example, the area ratios.

Hereinafter, variations of the embodiment described above is described.

(Variation 1)

In the above embodiment, as an example without being limited thereto, detector 102 performs the exposure control processing on a first RGB image, and performs the white balance adjustment processing on a third RGB image generated through the exposure control processing. However, the exposure control processing need not be performed. For instance, the exposure control processing need not be performed as long as a first RGB image in which the occurrence of variations in the luminance distribution is decreased is obtained. For instance, variations in the luminance distribution of a first RGB image to be obtained may be decreased by performing illumination control to make imaging conditions constant.

(Variation 2)

In the above embodiment, the accumulation level is detected using the image data that has undergone the image processing (e.g., the exposure control processing and the white balance adjustment processing). However, a part or all of the image processing need not be performed. For instance, an HSV image may be generated from a first RGB image, and the accumulation levels may be detected using the HSV image.

(Variation 3)

In the above embodiment, the accumulation level is detected using the value of an HSV image. However, the accumulation level determination method is not limited to the method. For instance, the saturation or hue of the HSV image may be used, or a combination of value and at least one of saturation or hue may be used. For instance, an evaluation value calculated from value and at least one of saturation or hue may be compared with a threshold.

In addition, the accumulation level may be detected using an RGB image. For instance, the R value of the RGB image may be used, and a combination of the R value and at least one of the G value or B value of the RGB image may be used. For instance, an evaluation value calculated from the R value and at least one of the G value or the B value may be compared with a threshold.

(Variation 4)

In the above embodiment, as an example without being limited thereto, intraoral camera 10 transmits a first RGB image to portable terminal 70, and portable terminal 70 then performs processing on the first RGB image. The first RGB image may be transmitted to cloud server 80, and cloud server 80 may then perform the processing and transmit the second RGB image or the fourth RGB image generated as a result of the processing to portable terminal 70. In this case, the first RGB image may be transmitted from intraoral camera 10 to cloud server 80 without via portable terminal 70 or may be transmitted from intraoral camera 10 to cloud server 80 via portable terminal 70.

(Variation 5)

In the above embodiment, the accumulation level is detected using an HSV image. However, the accumulation level determination method is not limited to the method. For instance, an HSL image may be used instead of an HSV image.

For instance, in the third image processing described above, detector 102 generates an HSL image by converting the color space of a second RGB image into an HSL space. Then, detector 102 identifies, as a dental plaque area, a specific pixel area in which one or more fifth pixels among the fifth pixels of the HSL image are located, the one or more fifth pixels satisfying at least one of the following conditions: the saturation is within a fourth predetermined range; the hue is within a fifth predetermined range; and the luminance is within a sixth predetermined range.

In addition, in the processing for detecting the concentration distribution of the fluorescent substance, which is described above, detector 102 detects the concentration distribution of the fluorescent substance by using the HSL image. Specifically, detector 102 detects the concentration distribution of the fluorescent substance by using the value of luminance L of the HSL image.

Here, an HSL color space (also referred to as an HLS color space) is a color space made up of the three components: H (hue), S (saturation), and L (luminance), and is obtained through nonlinear transformation of an RGB color space.

When the largest value among the values of R, G, and B is defined as MAX and the smallest value among the values is defined as MIN, H (hue) of an HSL image is calculated using the above Expression 5. In addition, L (luminance) is calculated using the following Expression 17. S (saturation) is calculated using the following Expression 18 when a cylindrical model is used. S (saturation) is calculated using the following Expression 19 when a biconical model is used.

[Math. 6]

$$L = \frac{MAX + MIN}{2} \quad \text{(Expression 17)}$$

$$S = \frac{MAX - MIN}{1 - |MAX + MIN - 1|} \quad \text{(Expression 18)}$$

$$S = MAX - MIN \quad \text{(Expression 19)}$$

Thus, in the HSL space, H, S, and L are calculated from MAX (max(R, G, B)) and MIN (min(R, G, B)). In the fluorescent area of dental plaque, when MIN = k × MAX, k = around 0 to 0.3.

FIG. 13 illustrates a relationship between the fluorescence intensity, MIN, and k. FIG. 14 illustrates a relationship between the fluorescence intensity, S (saturation), and k. FIG. 15 illustrates a relationship between the fluorescence intensity, L (luminance), and k. It should be noted that the example is an instance where the cylindrical model is used.

The features of k = 0 to 0.3 indicated by the continuous lines in FIGS. 13 to 15 correspond to the features of the fluorescent area of dental plaque. In addition, as illustrated in FIG. 14, when k = 0 to 0.3, S = 0.5 to 1. In addition, as illustrated in FIG. 15, when k = 0 to 0.3, L = 0 to 0.65.

Thus, when S is within the range of 0.5 to 1 in detecting dental plaque, L linearly changes in the range of 0 to 0.65. Thus, the value of L is proportional to the amount of porphyrin. Accordingly, the concentration distribution of the fluorescent substance (contents per unit area of the fluorescent substance contained in dental plaque and a dental calculus adhering to a tooth) can be detected on the basis of the value of L.

(Variation 6)

In the above explanation, the example of using blue-light blocking filter 20 is provided as a method of generating a first RGB image in which a blue-light region is at least partially attenuated from reflected light and fluorescence from a tooth, dental plaque, and a dental calculus inside an oral cavity that are being irradiated with irradiation light of a predetermined wavelength that excites a fluorescent substance contained in the dental plaque and the dental calculus. However, the blue-light region may be at least partially attenuated through signal processing without using blue-light blocking filter 20.

FIG. 16 illustrates an example of the pixel values of each image when a blue-light region is attenuated through signal processing. For instance, as illustrated in FIG. 16, detector 102 generates a fifth RGB image by performing blue-light blocking processing on a first RGB image that is image data obtained by image sensor 14. For instance, detector 102 generates the fifth RGB image by multiplying the blue pixel value of the first RGB image by gain = 0. It should be noted that the gain by which the blue pixel value is to be multiplied is not limited to 0, and detector 102 may multiply the blue pixel value by a predetermined gain smaller than 1. Alternatively, detector 102 may replace the blue pixel value with a predetermined value (for example, 0) or clip the blue pixel value to a predetermined value or lower.

Detector 102 generates a third RGB image by performing the exposure control processing on the fifth RGB image generated in this manner. For instance, detector 102 multiplies R, G, and B by the same gain to cause max(R, G, B) to be a predetermined level.

Next, detector 102 generates a second RGB image by performing the white balance adjustment processing on the third RGB image. For instance, detector 102 individually multiplies each of R and B by a gain to cause R and B to have the same level as G. It should be noted that when the blue-light blocking processing is performed, detector 102 need not multiply B by a gain in the white balance adjustment processing. In addition, the processing for detecting the concentration distribution of the fluorescent substance, which is described above, is performed using the second RGB image.

Even when the blue-light blocking processing is performed through the signal processing in this manner, it is possible to generate an image (fifth RGB image) in which the blue-light region is at least partially attenuated in the same way as blue-light blocking filter 20 is used.

It should be noted that the blue-light blocking processing may be performed on the second RGB image rather than on first RGB image. FIG. 17 illustrates an example of the pixel values of each image when the blue-light region is attenuated through the signal processing performed on the second RGB image.

In the example illustrated in FIG. 17, detector 102 generates a sixth RGB image by performing blue-light blocking processing similar to the above blue-light blocking processing, on the second RGB image that has undergone the white balance adjustment processing. The processing for detecting the concentration distribution of the fluorescent substance, which is described above, is performed using the sixth RGB image.

When the second RGB image illustrated in FIG. 16 and the sixth RGB image illustrated in FIG. 17 are compared, the second RGB image illustrated in FIG. 16 has a state closer to the state after the blue-light blocking is performed using an optical filter, the levels of remaining R and G in the second RGB image illustrated in FIG. 16 are higher, and the second RGB image illustrated in FIG. 16 is brighter.

It should be noted that the blue-light blocking processing may be digital processing or analog processing. In addition, in the example described above, detector 102 (portable terminal 70) performs the blue-light blocking processing. However, intraoral camera 10 may perform the blue-light blocking processing. It should be noted that in a general image sensor used in an RGB camera, the output order of the pixel values of R, G, and B is determined according to the RGB color filter array. Thus, the pixel value of B can be identified in the blue-light blocking processing.

As described above, the dental plaque detection device (e.g., portable terminal 70) according to the embodiment includes: obtainer 101 that obtains a first image (for example, the first RGB image) in which a blue-light region is at least partially attenuated from reflected light and fluorescence from a tooth, dental plaque, and a dental calculus inside an oral cavity that are being irradiated with irradiation light of a predetermined wavelength that excites a fluorescent substance contained in the dental plaque and the dental calculus; and detector 102 that detects, from the first image (for example, on the basis of the value of the intensity of fluorescence in the fluorescent substance in the first image), a content per unit area of the fluorescent substance (for example, the concentration distribution of the fluorescent substance) contained in the dental plaque and the dental calculus adhering to the tooth. As such, the dental plaque detection device can detect the condition of the tooth in detail since the dental plaque detection device can detect the content per unit area of the fluorescent substance contained in the dental plaque and the dental calculus adhering to the tooth. Furthermore, the dental plaque detection device can improve the detection accuracy of the content per unit area of the fluorescent substance contained in the dental plaque and the dental calculus adhering to the tooth, by using the first image in which the blue-light region is at least partially attenuated from the reflected light and the fluorescence from the tooth, the dental plaque, and the dental calculus inside the oral cavity that are being irradiated with the irradiation light of the predetermined wavelength that excites the fluorescent substance contained in the dental plaque and the dental calculus.

For instance, detector 102 generates an HSV image from the first image, and detects, from the value of the Value of the HSV image, the content per unit area of the fluorescent substance contained in the dental plaque and the dental calculus adhering to the tooth. Thus, the dental plaque detection device can detect the content per unit area of the fluorescent substance contained in the dental plaque and the dental calculus adhering to the tooth according to the value of the Value of the HSV image with high accuracy.

For instance, the first image is a first RGB image, and detector 102 generates a second RGB image by performing, on the first RGB image, image processing including first image processing. In the first image processing, a gain for each of at least two color components among the red component, the green component, and the blue component of an RGB image to be processed is adjusted so as to equalize the first red pixel average value of red pixel values, the first green pixel average value of green pixel values, and the first blue pixel average value of blue pixel values, the red pixel values, the green pixel values, and the blue pixel values being the pixel values of a plurality of first pixels making up the portion of the RGB image to be processed that corresponds to the tooth. Detector 102 generates the HSV image by converting the color space of the second RGB image into the HSV space.

As such, by performing the first image processing, the dental plaque detection device can adjust the white balance of the first RGB image showing a fluorescing tooth. In this way, the dental plaque detection device can generate the second RGB image in which a dental plaque area of a tooth that is a portion to which dental plaque is adhering is easily distinguished. Thus, the dental plaque detection device can improve the detection accuracy of the content per unit area of the fluorescent substance contained in the dental plaque and the dental calculus adhering to the tooth.

For instance, detector 102 identifies a specific pixel area where one or more fourth pixels among the plurality of fourth pixels of the HSV image are located, the one or more fourth pixels satisfying at least one of the following: the saturation is within a first predetermined range; the hue is within a second predetermined range; and the value is within a third predetermined range, and detector 102 detects, from the value of the Value of the specific pixel area in the HSV image, the content per unit area of the fluorescent substance contained in the dental plaque and the dental calculus adhering to the tooth. As such, the dental plaque detection device identifies a dental plaque area in a tooth image and then detects the content per unit area of the fluorescent substance contained in the dental plaque and the dental calculus adhering to the tooth, which enables the improvement in the detection accuracy of the content per unit area.

For instance, detector 102 generates an HSL image from the first image, and detects, from the value of the luminance of the HSL image, the content per unit area of the fluorescent substance contained in the dental plaque and the dental calculus adhering to the tooth. As such, the dental plaque detection device can detect the content per unit area of the fluorescent substance contained in the dental plaque and the dental calculus adhering to the tooth according to the value of the luminance of the HSL image with high accuracy.

For instance, the first image is a first RGB image, and detector 102 generates a second RGB image by performing, on the first RGB image, image processing including first image processing. In the first image processing, a gain for each of at least two color components among the red component, the green component, and the blue component of an RGB image to be processed is adjusted so as to equalize the first red pixel average value of red pixel values, the first green pixel average value of green pixel values, and the first blue pixel average value of blue pixel values, the red pixel values, the green pixel values, and the blue pixel values being the pixel values of a plurality of first pixels making up the portion of the RGB image to be processed that corresponds to the tooth. Detector 102 generates the HSL image by converting the color space of the second RGB image into an HSL space.

As such, by performing the first image processing, the dental plaque detection device can adjust the white balance of the first RGB image showing the fluorescing tooth. In this way, the dental plaque detection device can generate the second RGB image in which a dental plaque area of a tooth that is a portion to which dental plaque is adhering is easily distinguished. Thus, the dental plaque detection device can improve the detection accuracy of the content per unit area of the fluorescent substance contained in the dental plaque and the dental calculus adhering to the tooth.

For instance, detector 102 identifies a specific pixel area where one or more fifth pixels among the plurality of fifth pixels of the HSL image are located, the one or more fifth pixels satisfying at least one of the following: the saturation is within a fourth predetermined range; the hue is within a fifth predetermined range; and the luminance is within a sixth predetermined range, and detector 102 detects, from the value of the luminance of the specific pixel area, the content per unit area of the fluorescent substance contained in the dental plaque and the dental calculus adhering to the tooth. As such, the dental plaque detection device identifies a dental plaque area in a tooth image and then detects the content per unit area of the fluorescent substance contained in the dental plaque and the dental calculus adhering to the tooth, which enables the improvement in the detection accuracy of the content per unit area.

For instance, the fluorescent substance is porphyrin. For instance, detector 102 assigns one of three or more gradation levels to each of contents per unit area of the fluorescent substance contained in the dental plaque and the dental calculus adhering to the tooth, and detector 102 generates a third image (for example, the fourth RGB image) in which the contents per unit area shown in gradation are superimposed on a second image (for example, the first RGB image or the second RGB image) based on the first image. As such, for instance, by using the generated third image, it is possible to notify a user of the contents per unit area of the fluorescent substance contained in the dental plaque and the dental calculus adhering to the tooth.

For instance, the dental plaque detection device further includes: identifier 104 that identifies the type of a tooth imaged; and storage 105 that stores, in association with the type of the tooth identified, a content per unit area of a fluorescent substance contained in dental plaque and a dental calculus adhering to the tooth imaged, the content per unit area having been detected from the tooth imaged. As such, the dental plaque detection device can manage the content per unit area of the fluorescent substance contained in the dental plaque and the dental calculus adhering to each tooth.

The intraoral camera system according to the embodiment of the present disclosure is described above. However, the present disclosure is not limited to the embodiment.

For instance, in the examples described above, intraoral camera 10 mainly intended to capture an image of a tooth is used. However, intraoral camera 10 may be an oral care device including a camera. For instance, intraoral camera 10 may be, for example, a dental washer including a camera.

In addition, the processing units included in the intraoral camera system according to the embodiment are typically embodied as LSIs, which are integrated circuits. The processing units may be made as individual chips. Some or all of the processing units may be incorporated into one chip.

In addition, circuit integration may be achieved not only as an LSI but also as a dedicated circuit or a general-purpose processor. A field programmable gate array (FPGA), which can be programmed after manufacturing an LSI, or a reconfigurable processor in which the connections and settings of circuit cells inside an LSI are reconfigurable may be used.

In addition, in the embodiment, each of the structural elements may be dedicated hardware or may be achieved by executing a software program suitable for the structural element. The structural element may be achieved by a program executer, such as a CPU or a processor, reading and executing a software program stored in a recording medium, such as a hard disk or semiconductor memory.

In addition, the present disclosure may be embodied as, for example, an image display method to be performed by the intraoral camera system. In addition, the present disclosure may be embodied as the intraoral camera, the portable terminal, or the cloud server included in the intraoral camera system.

In addition, the functional block configuration illustrated in each block diagram is a mere example. Two or more functional blocks may be incorporated into one functional block. One functional block may be divided into more than one functional block. A part of the function may be transferred from one functional block to another functional block. In addition, the same hardware or software may process the functions of two or more functional blocks having similar functions in parallel or on a time-sharing basis.

In addition, the order in which the steps are performed in each flowchart is provided as an example to specifically explain the present disclosure. The steps may be performed in a different order. In addition, one or more of the steps may be performed simultaneously (in parallel) with another step.

The intraoral camera system(s) according to one aspect or aspects are described above on the basis of the embodiment. However, the present disclosure is not limited to the embodiment. Within the scope of the present disclosure, the one aspect or the aspects may include an embodiment obtained by making various changes envisioned by those skilled in the art to the embodiment and an embodiment obtained by combining some of the structural elements described in different embodiments.

[Industrial Applicability]

The present disclosure is applicable to intraoral camera systems.

[Reference Signs List]

| | |
|---|---|
| 10 | intraoral camera |
| 10a | head |
| 10b | handle |
| 10c | neck |
| 12 | imaging optical system |
| 12a | entry port |
| 14 | image sensor |
| 16 | lens |
| 18 | mirror |
| 20 | blue-light blocking filter |
| 24 | aperture |
| 26A | first LED |
| 26B | second LED |
| 26C | third LED |
| 26D | fourth LED |
| 28 | cover |
| 30 | composition adjustment mechanism |
| 32 | focus adjustment mechanism |
| 34 | case |
| 36, 40 | actuator |
| 38 | lens holder |

| 50 | central controller |
|---|---|
| 54 | LED controller |
| 56 | lens driver |
| 58 | wireless communication module |
| 60 | power supply controller |
| 62 | controller |
| 64 | memory |
| 66 | battery |
| 68 | coil |
| 69 | charger |
| 70 | portable terminal |
| 72 | touch screen |
| 80 | cloud server |
| 90 | position sensor |
| 101 | obtainer |
| 102 | detector |
| 103 | display |
| 104 | identifier |
| 105 | storage |
| 301 | tooth |
| 302 | gingiva |
| 303 | dental plaque |
| 304 | area |
| 305 | mature dental plaque |
| 306 | immature dental plaque |

**Claims**

1. A dental plaque detection device comprising:

   an obtainer that obtains a first image in which a blue-light region is at least partially attenuated from reflected light and fluorescence from a tooth, dental plaque, and a dental calculus inside an oral cavity that are being irradiated with irradiation light of a predetermined wavelength that excites a fluorescent substance contained in the dental plaque and the dental calculus; and
   a detector that detects, from the first image, a content per unit area of the fluorescent substance contained in the dental plaque and the dental calculus adhering to the tooth.

2. The dental plaque detection device according to claim 1, wherein
   the detector generates an HSV image from the first image, and detects, from a value of Value of the HSV image, the content per unit area of the fluorescent substance contained in the dental plaque and the dental calculus adhering to the tooth.

3. The dental plaque detection device according to claim 2, wherein

   the first image is a first RGB image,
   the detector generates a second RGB image by performing, on the first RGB image, image processing including first image processing,
   in the first image processing, a gain for each of at least two color components among a red component, a green component, and a blue component of an RGB image to be processed is adjusted so as to equalize a first red pixel average value of red pixel values, a first green pixel average value of green pixel values, and a first blue pixel average value of blue pixel values, the red pixel values, the green pixel values, and the blue pixel values being pixel values of a plurality of first pixels making up a portion of the RGB image to be processed that corresponds to the tooth, and
   the detector generates the HSV image by converting a color space of the second RGB image into an HSV space.

4. The dental plaque detection device according to claim 2 or 3, wherein

   the detector identifies a specific pixel area where one or more fourth pixels among a plurality of fourth pixels of the HSV image are located, the one or more fourth pixels satisfying at least one of the following:

Saturation is within a first predetermined range;
Hue is within a second predetermined range; and
Value is within a third predetermined range, and

the detector detects, from a value of the Value of the specific pixel area, the content per unit area of the fluorescent substance contained in the dental plaque and the dental calculus adhering to the tooth.

5. The dental plaque detection device according to claim 1, wherein
the detector generates an HSL image from the first image, and detects, from a value of luminance of the HSL image, the content per unit area of the fluorescent substance contained in the dental plaque and the dental calculus adhering to the tooth.

6. The dental plaque detection device according to claim 5, wherein

the first image is a first RGB image,
the detector generates a second RGB image by performing, on the first RGB image, image processing including first image processing,
in the first image processing, a gain for each of at least two color components among a red component, a green component, and a blue component of an RGB image to be processed is adjusted so as to equalize a first red pixel average value of red pixel values, a first green pixel average value of green pixel values, and a first blue pixel average value of blue pixel values, the red pixel values, the green pixel values, and the blue pixel values being pixel values of a plurality of first pixels making up a portion of the RGB image to be processed that corresponds to the tooth, and
the detector generates the HSL image by converting a color space of the second RGB image into an HSL space.

7. The dental plaque detection device according to claim 5 or 6, wherein

the detector identifies a specific pixel area where one or more fifth pixels among a plurality of fifth pixels of the HSL image are located, the one or more fifth pixels satisfying at least one of the following:

Saturation is within a fourth predetermined range;
Hue is within a fifth predetermined range; and
Luminance is within a sixth predetermined range, and

the detector detects, from a value of the Luminance of the specific pixel area, the content per unit area of the fluorescent substance contained in the dental plaque and the dental calculus adhering to the tooth.

8. The dental plaque detection device according to any one of claims 1 to 7, wherein
the fluorescent substance is porphyrin.

9. The dental plaque detection device according to any one of claims 1 to 8, wherein

the detector assigns one of three or more gradation levels to each of contents per unit area of the fluorescent substance contained in the dental plaque and the dental calculus adhering to the tooth, and
the detector generates a third image in which the contents per unit area shown in gradation are superimposed on a second image based on the first image.

10. The dental plaque detection device according to any one of claims 1 to 9, further comprising:

an identifier that identifies a type of a tooth imaged; and
storage that stores, in association with the type of the tooth identified, a content per unit area of a fluorescent substance contained in dental plaque and a dental calculus adhering to the tooth imaged, the content per unit area having been detected from the tooth imaged.

11. A dental plaque detection method comprising:

obtaining a first image in which a blue-light region is at least partially attenuated from reflected light and fluorescence from a tooth, dental plaque, and a dental calculus inside an oral cavity that are being irradiated with irradiation light of a predetermined wavelength that excites a fluorescent substance contained in the dental plaque and the dental calculus; and

detecting, from the first image, a content per unit area of the fluorescent substance contained in the dental plaque and the dental calculus adhering to the tooth.

12. A program for causing a computer to execute the dental plaque detection method according to claim 11.

FIG. 1

FIG. 2

# FIG. 3

EP 4 725 389 A1

# FIG. 4

```
┌─────────────────┐                              ┌─────────────────┐
│ Intraoral camera│ ～10                         │Portable terminal│ ～70
└─────────────────┘                              └─────────────────┘
        │                                                 │
        │ ～S101                                          │
 ┌──────────────┐                                         │
 │Capture image │                                         │
 └──────────────┘                                         │
        │                                                 │
        │        Image data       ～S102                  │
        │────────────────────────────────────────────────▶│
        │                                                 │
        │                                          ～S103  │
        │                                  ┌──────────────────────┐
        │                                  │ Perform image        │
        │                                  │ processing           │
        │                                  └──────────────────────┘
        │                                                 │
        │                                          ～S104  │
        │                                  ┌──────────────────────┐
        │                                  │ Detect concentration │
        │                                  │ distribution of      │
        │                                  │ fluorescent          │
        │                                  │ substance            │
        │                                  └──────────────────────┘
        │                                                 │
        │                                          ～S105  │
        │                                  ┌──────────────────────┐
        │                                  │ Generate image       │
        │                                  └──────────────────────┘
        │                                                 │
        │                                          ～S106  │
        │                                  ┌──────────────────────┐
        │                                  │ Display              │
        │                                  └──────────────────────┘
        │                                                 │
```

FIG. 5

FIG. 6

302

304

303

301

FIG. 7

302

301

305

303

306

# FIG. 8

Luminance $E_{\lambda 1}$ of LED light

Fluorescence (illuminance) $E_{\lambda 2}$ (D)

Attenuate

Illuminance $E_{\lambda 1}$ (D) of LED light

Excite

Fluorescence (luminance) $E_{\lambda 2}$

Attenuate

D

# FIG. 9

Fluorescence (illuminance) $E_{\lambda 2\_t}$ (D0)

D0

# FIG. 10

```
                    ┌──────────────┐
                    │    Start     │
                    └──────┬───────┘
                           │
                           ▼                    S121
                  ╱─────────────────╲   No
                 ╱  Value is less than ╲──────────────┐
                 ╲   first threshold   ╱               │
                  ╲─────────────────╱                  ▼              S123
                           │ Yes              ╱─────────────────╲   No
                           │                 ╱  Value is less than ╲──────────┐
                           │                 ╲   second threshold  ╱          │
                           │                  ╲─────────────────╱             │
                           │                          │ Yes                   │
                           ▼          S122            ▼          S124         ▼          S125
                 ┌──────────────────┐  ┌──────────────────────┐  ┌──────────────────────┐
                 │ Accumulation     │  │ Accumulation level 1 │  │ Accumulation level 2 │
                 │ level 0          │  │ (immature dental     │  │ (mature dental       │
                 │ (no dental       │  │  plaque)             │  │  plaque)             │
                 │  plaque)         │  └──────────┬───────────┘  └──────────┬───────────┘
                 └────────┬─────────┘             │                         │
                          │◄─────────────────────┴─────────────────────────┘
                          ▼
                 ┌──────────────┐
                 │     End      │
                 └──────────────┘
```

FIG. 11

306    305

302

301

FIG. 12

305

302

301

## FIG. 13

## FIG. 14

FIG. 15

# FIG. 16

Image data
(first RGB image)

After blue-light
blocking processing
(fifth RGB image)

After exposure
control processing
(third RGB image)

After white balance
adjustment processing
(second RGB image)

R  G  B                R  G  B                R  G  B                R  G  B

# FIG. 17

Image data
(first RGB image)

After exposure
control processing
(third RGB image)

After white balance
adjustment processing
(second RGB image)

After blue-light
blocking processing
(sixth RGB image)

R  G  B          R  G  B          R  G  B          R  G  B

## INTERNATIONAL SEARCH REPORT

| International application No. |
| --- |
| **PCT/JP2024/020178** |

**A. CLASSIFICATION OF SUBJECT MATTER**

*A61B 1/24*(2006.01)i; *A61B 1/00*(2006.01)i; *A61C 19/04*(2006.01)i; *A61C 19/06*(2006.01)i
FI: A61B1/24; A61B1/00 511; A61C19/06 A; A61C19/04 Z

According to International Patent Classification (IPC) or to both national classification and IPC

**B. FIELDS SEARCHED**

Minimum documentation searched (classification system followed by classification symbols)

A61B1/24; A61B1/00; A61C19/04; A61C19/06

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y | JP 2014-133029 A (PANASONIC CORPORATION) 24 July 2014 (2014-07-24) | 1-2, 4-5, 7-12 |
| | paragraphs [0023]-[0106] | |
| A | paragraphs [0023]-[0106] | 3, 6 |
| Y | JP 2014-4329 A (SONY CORPORATION) 16 January 2014 (2014-01-16) | 1-2, 4-5, 7-12 |
| | paragraph [0038] | |
| Y | WO 2016/140199 A1 (CITIZEN WATCH CO., LTD.) 09 September 2016 (2016-09-09) | 1-2, 4-5, 7-12 |
| | paragraphs [0003], [0046] | |

☐ Further documents are listed in the continuation of Box C.  ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **25 June 2024** | **09 July 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT
### Information on patent family members

| Patent document cited in search report | | | Publication date (day/month/year) | Patent family member(s) | | | Publication date (day/month/year) |
|---|---|---|---|---|---|---|---|
| JP | 2014-133029 | A | 24 July 2014 | (Family: none) | | | |
| JP | 2014-4329 | A | 16 January 2014 | US paragraph [0125] | 2013/0323673 | A1 | |
| | | | | CN | 103445877 | A | |
| WO | 2016/140199 | A1 | 09 September 2016 | US paragraphs [0003], [0071] | 2018/0035896 | A1 | |
| | | | | EP | 3267182 | A1 | |
| | | | | CN | 107430072 | A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013248220 A